# EUROPEAN PATENT SPECIFICATION

(11) **EP 0 818 467 B1**
(45) Date of publication and mention of the grant of the patent: **02.11.2000**
(21) Application number: 97111868.2
(22) Date of filing: 11.07.1997
(51) Int. Cl.: C07K 17/02, C07K 14/705, C12N 9/12, G01N 33/543

(54) **Aligned peptide array and a rational and rapid method for the detection of a binding or interaction site of a protein by using the same**
Ausgerichtete Peptid-Anordnung und ein rationelles und schnelles Verfahren zum Nachweis einer Bindung oder einer Wechselwirkungsstelle eines Proteins mittels deren
Réseau peptidique aligné et procédé rapide et rationnel de dépistage d'une liaison ou d'un site d'interaction d'une protéine en utilisant celui-ci

(30) Priority: 12.07.1996 JP 18314096
(43) Date of publication of application: 14.01.1998
(73) Proprietor: NEC CORPORATION, Tokyo (JP)
(72) Inventor: Miwa, Johji c/o NEC Corporation, Tokyo (JP); Siddiqui, Shahid Saeed, Kitayama-cho Toyohashi-shi Aichi (JP)
(74) Representative: Glawe, Delfs, Moll & Partner

(56) References cited:
- US-A- 5 318 679
- S P A FODOR ET AL. : "Light-directed, spatially addressable parallel chemical synthesis" SCIENCE., vol. 251, 15 February 1991, LANCASTER, PA US, pages 767-773, XP000486899
- M A ATOR ET AL.: "Immobilized peptide arrays: a new technology for the characterization of protease function" PEPTIDES. CHEMISTRY, STRUCTURE AND BIOLOGY. PROCEEDINGS OF THE 13TH AMERICAN PEPTIDE SYMPOSIUM, JUNE 20-25, 1993, EDMONTON, ALBERTA, CANADA , 1994, LEIDEN, ESCOM, pages 1012-101014, XP002055368
- P BORNSTEIN ET AL.: "The limited cleavage of native collagen with chymotrypsin, trypsin and cyanogen bromide " BIOCHEMISTRY., vol. 5, no. 12, December 1966, EASTON, PA US, pages 3803-3812, XP002055375
- J A JAMES & J B HARLEY: "Linear epitope mapping of an Sm B/B' polypeptide" JOURNAL OF IMMUNOLOGY, vol. 148, no. 7, 1 April 1992, BALTIMORE US, pages 2074-2079, XP002055380

## Description

### BACKGROUND OF THE INVENTION

### 1. Field of the Invention

This invention relates to aligned peptides and their immobilized preparations. It also relates to rational and rapid methods for detecting the binding or interaction site of a protein with a ligand therefor (i.e., a substance which binds to or interacts with the protein), or for the detection of such a ligand, by using the same, to methods for the modification or design of a protein or a ligand by utilizing information on a site so detected, and to immunoassay methods.

### 2. Description of the Related Art

Conventionally, there has been no method for rationally, rapidly, and and systematically detecting the binding or interaction site of a protein with a ligand therefor.

For example, the conventional detection of a specific protein by using an antigen-antibody reaction is generally carried out on the basis of the presence of reaction with the protein as a whole, whether the antibody used is polyclonal or monoclonal. In this case, therefore, (1) the detection of the reaction does not necessarily lead to the detection of the binding site and, moreover, (2) it cannot be known how many binding sites actually exist. Moreover, in the case of detection by western. blotting or detection in situ (e.g., in cells or tissues) by immune antibody techniques, (3) it is frequently impossible to know whether the binding protein is the specifically desired protein to be detected or not. J. Immunology 148 (7), pp. 2074 to 2079 (1992) discloses a peptide array derived from Sm B/B' polypeptide, with segments of suitable length and suitable frame (octapeptide). Overlapping octapeptides of this polypeptide are prepared to study the interaction sites.

The present invention is based on an entirely new conception and there is no existing technique that is directly comparable to it.

As described above, because there has been no method for the direct rational and rapid detection of the binding or interaction site of a protein with a ligand therefor, the modification of a protein or ligand and the design of a new protein or ligand has depended on chance. Accordingly, such tasks have been carried out by relying on mere chance or the "intuition" of an expert.

### SUMMARY OF THE INVENTION

An object of the present invention is to solve the above-described problems by providing a method for rationally, rapidly, systematically, and conveniently detecting the binding or interaction site of a protein with a ligand therefor. Another object of the present invention is to provide a rational and rapid method for detecting a ligand by using the above method. Still another object of the present invention is to provide rational and rapid methods for the modification or design of a protein or a ligand by utilizing information obtained by the above described methods, as well as by immunoassay methods.

To accomplish the above objectives, the present inventors have made various investigations and have now completed the present invention. The present invention is defined in the claims.

According to a first aspect of the present invention, there is provided an aligned peptide array comprising, as separate elements, a plurality of peptide segments obtained by dividing the amino acid sequence of a protein into nonoverlapping sequence segments of equal length and of any suitable frame and synthesizing peptides on the basis of the sequence segments, wherein the peptide segments maintain each position thereof.

According to a second aspect of the present invention, there is provided an aligned peptide array comprising, as separate elements, a plurality of peptide segments obtained by dividing the amino acid sequence of a protein into nonoverlapping sequence segments of equal length and of any suitable frame and synthesizing peptides on the basis of the sequence segments, the amino acid sequences of the peptide segments expressing the amino acid sequence of said protein, wherein the peptide segments maintain each position thereof.

According to a third aspect of the present invention, there is provided an immobilized aligned peptide array preparation obtained by immobilizing the aligned peptide array in accordance with the first or second aspect of the present invention.

According to a fourth aspect of the present invention, there is provided a rational and rapid method for the detection of a binding or interaction site of a protein which comprises detecting the binding or interaction site of any suitable protein or a specific protein with a ligand therefor by using the aligned peptide array in accordance with the first or second aspect of the present invention or an immobilized aligned peptide array preparation derived therefrom.

According to a fifth aspect of the present invention, there is provided a rational and rapid method for the detection of a ligand for a protein which comprises a step using the detection method in accordance with the fourth aspect of the present invention.

According to a sixth aspect of the present invention, there is provided a rational and rapid method for the modification of a protein which comprises a step using the detection method in accordance with the fourth aspect of the present invention.

According to a seventh aspect of the present invention, there is provided a rational and rapid method for the design of a protein which comprises a step using the detection method in accordance with the fourth aspect of the present invention.

According to an eighth aspect of the present invention, there is provided a rational and rapid method for the modification of a ligand detected by the detection method in accordance with the fifth aspect of the present invention which comprises a step utilizing the information on a binding or interaction site of a protein as obtained by the detection method in accordance with the fourth aspect of the present invention.

According to a ninth aspect of the present invention, there is provided a rational and rapid method for the design of a ligand detected by the detection method in accordance with the fifth aspect of the present invention which comprises a step utilizing the information on a binding or interaction site of a protein as obtained by the detection method in accordance with the fourth aspect of the present invention.

According to a tenth aspect of the present invention, there is provided a rational and rapid immunoassay method which comprises using the aligned peptide array in accordance with the first or second aspect of the present invention or an immobilized aligned peptide array preparation derived therefrom.

According to the present invention, the binding or interaction site of a protein with a ligand therefor can be detected, rationally, rapidly, systematically, and conveniently.

The detection of a ligand for a specific protein or its binding site as described in Example 2 of the present invention is not only indispensable for identification and understanding of the mode of action of the protein at a molecular level, but also very useful in the design of a protein having new properties by modifying its binding site as described in Example 3. In Example 3, for instance, a protein having the ability to combine with a phorbol ester was converted into a protein not having that ability. If this conception is extended, the reverse conversion will also be possible. Moreover, if the phorbol ester is taken as a ligand, this procedure may be applied to the detection of any desired protein and a ligand therefor.

Furthermore, if a ligand-binding or interaction site can be identified and understood at a molecular level, it will become easier to design or modify the ligand. As a result, it will become correspondingly easier to design, for example, a chemical agent or physiologically active substance that can act directly on a specific protein by recognizing it as a ligand.

Thus, the present invention also serves to create a protein or ligand having a more desirable function by modifying or designing the ligand-binding or ligand-interaction site of the protein or by modifying or designing the ligand.

Moreover, the present invention makes it possible to search for unknown ligands for any desired protein rationally, rapidly, systematically, deliberately, exhaustively, and economically.

Furthermore, the present invention can provide an excellent immunoassay method because, on the basis of an antigen-antibody reaction, the reacting (i.e., the binding or interacting) peptide segment(s) can readily be observed with the naked eye, a microscope, or other detection devices.

### DETAILED DESCRIPTION OF THE PREFERRED EMBODIMENTS

In the present invention, the amino acid sequence of a protein is divided into non-overlapping sequence segments of equal length and of any suitable overlapping frame, and peptides corresponding to the respective sequence segments (hereinafter referred to as "peptide segments") are synthesized. The individual peptide segments so synthesized are arranged in suitable order, for example, starting from a terminus (i.e., the amino or carboxyl terminus) of the protein. Then, they are immobilized on a suitable substance, or made into solutions and held in a suitable container. This plurality of suitably arranged peptide segments is called an aligned peptide array or a peptall or PEPTALL.

For example, an aligned peptide array can be formed by suitably arranging peptide segments so as to give the amino acid sequence of a protein as shown in the following formula (I). In this formula, the peptide units separated by "//" are independent peptide segments. Although each peptide segment consists of 10 amino acids in this example, the number of amino acids and the frame of peptide segments in a protein may be arbitrarily chosen.

MAQAENACRL//KLLRADVPVD//LLPAGCSATD//LQPAVNVKEK//IEVNGESRLV//
QKKKTLYPEW//EKCWDTAVAE//RILQIVLMFN//QPVVEATMRL//EDIISKCKSD
(SEQ ID NO:1-10)                                    (I)

At present, if each peptide segment consists of up to several tens of amino acids, several tens of peptide segments can be concurrently synthesized in a short period of time by use of a single peptide synthesizer. Moreover, there are an increasing number of studies demonstrating that a protein may be regarded as an aggregate consisting of several to several tens of relatively short functional peptides.

Substances suitable for the purpose of immobilizing the aligned peptide array of the present invention include membranes such as membrane filters. They also include gels in plate form, such as agar and polyacrylamide. Specific examples of the gels in plate form are agar media for the cultivation of bacteria which are placed in containers such as Petri dishes. Moreover, the aligned peptide array of the present invention may also be made by placing its peptide segments in a container which permits them to exist separately from each other. Specifically, the aligned peptide array may be made by preparing solutions containing its peptide segments and placing them in microtiter wells so that they exist separately from each other. Furthermore, the aligned peptide array of the present invention may be made in the form of a microchip or microdevice comprising an integrated circuit on which the aligned peptide array is arranged and immobilized.

According to the present invention, based on the basic conception that the amino acid sequence of a protein is divided into non-overlapping peptide units of equal length, a variety of detailed information about peptide segments corresponding to the peptide units can be acquired by carrying out various detailed experiments (including testing and designing) on an aligned peptide array comprising the regularly arranged peptide segments. In other words, this means that detailed information about any desired portion of the original protein can be obtained. Thus, the knowledge and information required for the modification of the original protein or the design of a new protein can be obtained rationally,rapidly, analytically, systematically, and easily. Moreover, a ligand for any desired protein can be detected, and knowledge and information on the ligand-binding or ligand-interaction site of the protein can be obtained rationally, rapidly, analytically, and systematically. Thus, the knowledge and information required for the modification of the ligand or the novel designing of a ligand can be obtained rationally, rapidly, analytically, and systematically.

The term "ligand" as used herein denotes any substance that binds to or interacts with proteins or any other molecules. Examples thereof include antibodies; chemical agents such as pharmaceutical drugs, agricultural chemicals, and insecticides; physiologically active substances such as toxins, pheromones, and hormones; and biological substances such as other proteins, nucleic acids (e.g., DNA and RNA), carbohydrates, and lipids.

Obviously, the concept used for the present invention can be applied to make arrays for any molecules for detection of any binding or interacting molecules.

The present invention can also be applied to immunoassay methods. Examples thereof include enzyme immunoassay typified by ELISA, viroimmunoassay, metalloimmunoassay, fluoroimmunoassay, and radioimmunoassay.

In these immunoassay methods, an antibody may first be modified with an appropriate substance (i.e., an enzyme, bacteriophage, metal, fluorescent substance, or radioactive isotope) and then reacted with an aligned peptide to detect its antibody-binding or interaction site. Alternatively, an antibody may first be reacted with an aligned peptide and then modified with such a substance prior to detection. The purpose of the modification is to facilitate the observation of the site of reaction with the antibody (i.e., the binding or interaction site). That is, instead of detecting the antibody directly, the antibody is detected with detection sensitivity enhanced by the use of such an appropriate modifier.

The present invention is further illustrated by the following examples. These examples, however, are not to be construed as limiting the scope of the invention.

### Example 1

The present invention is explained in connection with an example in which it is applied to the detection of the binding site of a specific protein with an antibody against the protein (i.e., the antibody recognition site of the protein). In this example, it was tried to detect the binding site of tubulin, which is a protein constituting microtubules, with an antibody against it (i.e., anti-tubulin antibody).

First of all, the amino acid sequence of alpha 3-tubulin derived from a nematode was divided into 45 sequence segments each consisting of 10 amino acids (except the final sequence segment consisting of 12 amino acids), as shown in the following formula (II).

(1)QREVISIHIG(2)QAGVQIGNAC(3)WELYCLEHGI(4)QPDGQMPSDK
(5)SLGGSDDSFS(6)TFFSETGSGR(7)HVPRAVNVDL(8)EPTVIDEIRT
(9)GTYRSLFHPE(10)QLITGKEDAA(11)NNYARGHYTI(12)GKEEIIDLTL
(13)DRIRRLADNC(14)TGLQGFLVFH(15)SFGGGTGSGF(16)TSLLNERLSV
(17)DYGKKAKLEF(18)SIYPAPQVST(19)AVVEPYNSIL(20)TTHTTLEHSD
(21)CSFNVDNEAI(22)YDICRRNLDI(23)ERPSYTNLNR(24)LIGQIVSSIT
(25)ASLRFDGALN(26)VDLTEFQTNL(27)VPYPRIHFPL(28)ATFSPVISAE
(29)KAYHEQLSVA(30)EITNNCFEPH(31)NQNVKCDPRH(32)RGDVVPKDVN
(33)RGDVVPKDVN(34)AAIATIKTKR(35)SIQFVDWCPT(36)GFKYVGINYQ

(37)PPTVVPGGDL(38)AKVPRAVCML(39)SNTTAIAEAW(40)ARLDHKFDLM
(41)YAKRAFVHWY(42)VGEGMEEGEF(43)SEAREDLAAL(44)EDKYEEVGVD
(45)SMEDNGEEGDEY (SEQ ID NO:11-55)                    (II)

Next, peptide segments were synthesized according to the amino acid sequences of the respective sequence segments. These peptide segments were arranged and immobilized on a membrane filter to prepare an aligned peptide array membrane.

This aligned peptide array membrane was first reacted with anti-acetylated tubulin antibody, and then with a secondary antibody comprising mouse anti-rabbit IgG tagged with horseradish peroxidase. Thereafter, the reaction spot was identified by staining the aligned peptide array membrane by means of a color development reaction.

In this example, color development was observed in the peptide segments corresponding to the fourth and fifth sequence segments of formula (II).

It can be concluded from the above results that the binding site of alpha 3-tubulin with the anti-acetylated tubulin antibody used in this example lies in the amino acid sequence QPDGQMPSDKSLGGSDDSFS.

### Example 2

First of all, an amino acid sequence corresponding to a portion of the regulatory region of protein kinase C (PKC) was divided as shown in the following formula (III), and the corresponding peptide segments were synthesized. Then, an aligned peptide array membrane was prepared in the same manner as given in Example 1.

(1)VHEIRGHQFVATFFR(2)QPHFCSLCSDFMWGL(3)NKQGYQCQLCSAAVH
(4)KKCHEKVIMQCPGSA(5)KNTKETMALKERFKV(6)DIPHRFKTYNFKSPT
(7)FCDHCGSMLYGLFKQ(8)GLRCEVCNVACHHKC(9)ERLMSNLCGVNQKQL
(SEQ ID NO:56-64) (III)

The aligned peptide array membrane was reacted with a phorbol ester labeled with tritium (³H) and then brought into contact with a photographic film. Thus, the reaction spot was identified by autoradiography.

In this example, exposure to radiation was observed at the position of the peptide segment corresponding to the third sequence segment of formula (III). This sequence segment corresponds to a "zinc finger-like" sequence that has conventionally been presumed to be a phorbol ester-binding site.

### Example 3

In the third peptide segment detected in Example 2, one amino acid was replaced as shown in the following formula (IV). Excepting this modification, detection was carried out in the same manner as in Example 2.

NKQGYQCQLCSAAVH → NKQEYQCQLCSAAVH (SEQ ID NO:65) (IV)

As a result, no exposure to radiation was observed at the position of the third peptide segment, in which one amino acid had been replaced. This fact can be interpreted to mean that, in consequence of the replacement of G (glycine) in the third peptide segment by E (glutamate), the phorbol ester acting as a ligand lost its ability to bind to this region.

### SEQUENCE LISTING

SEQ ID NO:1
   SEQUENCE LENGTH:10
   SEQUENCE TYPE:amino acid
   MOLECULE TYPE:peptide
   SEQUENCE: 
Met Ala Gln Ala Glu Asn Ala Cys Arg Leu
1               5                   10
SEQ ID NO:2
   SEQUENCE LENGTH:10
   SEQUENCE TYPE:amino acid
   MOLECULE TYPE:peptide
   SEQUENCE: 
Lys Leu Leu Arg Ala Asp Val Pro Val Asp
1               5                   10
SEQ ID NO:3
   SEQUENCE LENGTH:10
   SEQUENCE TYPE:amino acid
   MOLECULE TYPE:peptide
   SEQUENCE: 
Leu Leu Pro Ala Gly Cys Ser Ala Thr Asp
1               5                   10
SEQ ID NO:4
   SEQUENCE LENGTH:10
   SEQUENCE TYPE:amino acid
   MOLECULE TYPE:peptide
   SEQUENCE: 
Leu Gln Pro Ala Val Asn Val Lys Glu Lys
1               5                   10
SEQ ID NO:5
   SEQUENCE LENGTH:10
   SEQUENCE TYPE:amino acid
   MOLECULE TYPE:peptide
   SEQUENCE: 
Ile Glu Val Asn Gly Glu Ser Arg Leu Val
1               5                   10
SEQ ID NO:6
   SEQUENCE LENGTH:10
   SEQUENCE TYPE:amino acid
   MOLECULE TYPE:peptide
   SEQUENCE: 
Gln Lys Lys Lys Thr Leu Tyr Pro Glu Trp
1               5                   10
SEQ ID NO:7
   SEQUENCE LENGTH:10
   SEQUENCE TYPE:amino acid
   MOLECULE TYPE:peptide
   SEQUENCE: 
Glu Lys Cys Trp Asp Thr Ala Val Ala Glu
1               5                   10
SEQ ID NO:8
   SEQUENCE LENGTH:10
   SEQUENCE TYPE:amino acid
   MOLECULE TYPE:peptide
   SEQUENCE: 
Arg Ile Leu Gln Ile Val Leu Met Phe Asn
1               5                   10
SEQ ID NO:9
   SEQUENCE LENGTH:10
   SEQUENCE TYPE:amino acid
   MOLECULE TYPE:peptide
   SEQUENCE: 
Gln Pro Val Val Glu Ala Thr Met Arg Leu
1               5                   10
SEQ ID NO:10
   SEQUENCE LENGTH:10
   SEQUENCE TYPE:amino acid
   MOLECULE TYPE:peptide
   SEQUENCE: 
Glu Asp Ile Ile Ser Lys Cys Lys Ser Asp
1               5                   10
SEQ ID NO:11
   SEQUENCE LENGTH:10
   SEQUENCE TYPE:amino acid
   MOLECULE TYPE:peptide
   SEQUENCE: 
Gln Arg Glu Val Ile Ser Ile His Ile Gly
1               5                   10
SEQ ID NO:12
   SEQUENCE LENGTH:10
   SEQUENCE TYPE:amino acid
   MOLECULE TYPE:peptide
   SEQUENCE: 
Gln Ala Gly Val Gln Ile Gly Asn Ala Cys
1               5                   10
SEQ ID NO:13
   SEQUENCE LENGTH:10
   SEQUENCE TYPE:amino acid
   MOLECULE TYPE:peptide
   SEQUENCE: 
Trp Glu Leu Tyr Cys Leu Glu His Gly Ile
1               5                   10
SEQ ID NO:14
   SEQUENCE LENGTH:10
   SEQUENCE TYPE:amino acid
   MOLECULE TYPE:peptide
   SEQUENCE: 
Gln Pro Asp Gly Gln Met Pro Ser Asp Lys
1               5                   10
SEQ ID NO:15
   SEQUENCE LENGTH:10
   SEQUENCE TYPE:amino acid
   MOLECULE TYPE:peptide
   SEQUENCE: 
Ser Leu Gly Gly Ser Asp Asp Ser Phe Ser
1               5                   10
SEQ ID NO:16
   SEQUENCE LENGTH:10
   SEQUENCE TYPE:amino acid
   MOLECULE TYPE:peptide
   SEQUENCE: 
Thr Phe Phe Ser Glu Thr Gly Ser Gly Arg
1               5                   10
SEQ ID NO:17
   SEQUENCE LENGTH:10
   SEQUENCE TYPE:amino acid
   MOLECULE TYPE:peptide
   SEQUENCE: 
His Val Pro Arg Ala Val Asn Val Asp Leu
1               5                   10
SEQ ID NO:18
   SEQUENCE LENGTH:10
   SEQUENCE TYPE:amino acid
   MOLECULE TYPE:peptide
   SEQUENCE: 
Glu Pro Thr Val Ile Asp Glu Ile Arg Thr
1               5                   10
SEQ ID NO:19
   SEQUENCE LENGTH:10
   SEQUENCE TYPE:amino acid
   MOLECULE TYPE:peptide
   SEQUENCE: 
Gly Thr Tyr Arg Ser Leu Phe His Pro Glu
1               5                   10
SEQ ID NO:20
   SEQUENCE LENGTH:10
   SEQUENCE TYPE:amino acid
   MOLECULE TYPE:peptide
   SEQUENCE: 
Gln Leu Ile Thr Gly Lys Glu Asp Ala Ala
1               5                   10
SEQ ID NO:21
   SEQUENCE LENGTH:10
   SEQUENCE TYPE:amino acid
   MOLECULE TYPE:peptide
   SEQUENCE: 
Asn Asn Tyr Ala Arg Gly His Tyr Thr Ile
1               5                   10
SEQ ID NO:22
   SEQUENCE LENGTH:10
   SEQUENCE TYPE:amino acid
   MOLECULE TYPE:peptide
   SEQUENCE: 
Gly Lys Glu Glu Ile Ile Asp Leu Thr Leu
1               5                   10
SEQ ID NO:23
   SEQUENCE LENGTH:10
   SEQUENCE TYPE:amino acid
   MOLECULE TYPE:peptide
   SEQUENCE: 
Asp Arg Ile Arg Arg Leu Ala Asp Asn Cys
1               5                   10
SEQ ID NO:24
   SEQUENCE LENGTH:10
   SEQUENCE TYPE:amino acid
   MOLECULE TYPE:peptide
   SEQUENCE: 
Thr Gly Leu Gln Gly Phe Leu Val Phe His
1               5                   10
SEQ ID NO:25
   SEQUENCE LENGTH:10
   SEQUENCE TYPE:amino acid
   MOLECULE TYPE:peptide
   SEQUENCE: 
Ser Phe Gly Gly Gly Thr Gly Ser Gly Phe
1               5                   10
SEQ ID NO:26
   SEQUENCE LENGTH:10
   SEQUENCE TYPE:amino acid
   MOLECULE TYPE:peptide
   SEQUENCE: 
Thr Ser Leu Leu Asn Glu Arg Leu Ser Val
1               5                   10
SEQ ID NO:27
   SEQUENCE LENGTH:10
   SEQUENCE TYPE:amino acid
   MOLECULE TYPE:peptide
   SEQUENCE: 
Asp Tyr Gly Lys Lys Ala Lys Leu Glu Phe
1               5                   10
SEQ ID NO:28
   SEQUENCE LENGTH:10
   SEQUENCE TYPE:amino acid
   MOLECULE TYPE:peptide
   SEQUENCE: 
Ser Ile Tyr Pro Ala Pro Gln Val Ser Thr
1               5                   10
SEQ ID NO:29
   SEQUENCE LENGTH:10
   SEQUENCE TYPE:amino acid
   MOLECULE TYPE:peptide
   SEQUENCE: 
Ala Val Val Glu Pro Tyr Asn Ser Ile Leu
1               5                   10
SEQ ID NO:30
   SEQUENCE LENGTH:10
   SEQUENCE TYPE:amino acid
   MOLECULE TYPE:peptide
   SEQUENCE: 
Thr Thr His Thr Thr Leu Glu His Ser Asp
1               5                   10
SEQ ID NO:31
   SEQUENCE LENGTH:10
   SEQUENCE TYPE:amino acid
   MOLECULE TYPE:peptide
   SEQUENCE: 
Cys Ser Phe Asn Val Asp Asn Glu Ala Ile
1               5                   10
SEQ ID NO:32
   SEQUENCE LENGTH:10
   SEQUENCE TYPE:amino acid
   MOLECULE TYPE:peptide
   SEQUENCE: 
Tyr Asp Ile Cys Arg Arg Asn Leu Asp Ile
1               5                   10
SEQ ID NO:33
   SEQUENCE LENGTH:10
   SEQUENCE TYPE:amino acid
   MOLECULE TYPE:peptide
   SEQUENCE: 
Glu Arg Pro Ser Tyr Thr Asn Leu Asn Arg
1               5                   10
SEQ ID NO:34
   SEQUENCE LENGTH:10
   SEQUENCE TYPE:amino acid
   MOLECULE TYPE:peptide
   SEQUENCE: 
Leu Ile Gly Gln Ile Val Ser Ser Ile Thr
1               5                   10
SEQ ID NO:35
   SEQUENCE LENGTH:10
   SEQUENCE TYPE:amino acid
   MOLECULE TYPE:peptide
   SEQUENCE: 
Ala Ser Leu Arg Phe Asp Gly Ala Leu Asn
1               5                   10
SEQ ID NO:36
   SEQUENCE LENGTH:10
   SEQUENCE TYPE:amino acid
   MOLECULE TYPE:peptide
   SEQUENCE: 
Val Asp Leu Thr Glu Phe Gln Thr Asn Leu
1               5                   10
SEQ ID NO:37
   SEQUENCE LENGTH:10
   SEQUENCE TYPE:amino acid
   MOLECULE TYPE:peptide
   SEQUENCE: 
Val Pro Tyr Pro Arg Ile His Phe Pro Leu
1               5                   10
SEQ ID NO:38
   SEQUENCE LENGTH:10
   SEQUENCE TYPE:amino acid
   MOLECULE TYPE:peptide
   SEQUENCE: 
Ala Thr Phe Ser Pro Val Ile Ser Ala Glu
1               5                   10
SEQ ID NO:39
   SEQUENCE LENGTH:10
   SEQUENCE TYPE:amino acid
   MOLECULE TYPE:peptide
   SEQUENCE: 
Lys Ala Tyr His Glu Gln Leu Ser Val Ala
1               5                   10
SEQ ID NO:40
   SEQUENCE LENGTH:10
   SEQUENCE TYPE:amino acid
   MOLECULE TYPE:peptide
   SEQUENCE: 
Glu Ile Thr Asn Asn Cys Phe Glu Pro His
1               5                   10
SEQ ID NO:41
   SEQUENCE LENGTH:10
   SEQUENCE TYPE:amino acid
   MOLECULE TYPE:peptide
   SEQUENCE: 
Asn Gln Asn Val Lys Cys Asp Pro Arg His
1               5                   10
SEQ ID NO:42
   SEQUENCE LENGTH:10
   SEQUENCE TYPE:amino acid
   MOLECULE TYPE:peptide
   SEQUENCE: 
Arg Gly Asp Val Val Pro Lys Asp Val Asn
1               5                   10
SEQ ID NO:43
   SEQUENCE LENGTH:10
   SEQUENCE TYPE:amino acid
   MOLECULE TYPE:peptide
   SEQUENCE: 
Arg Gly Asp Val Val Pro Lys Asp Val Asn
1               5                   10
SEQ ID NO:44
   SEQUENCE LENGTH:10
   SEQUENCE TYPE:amino acid
   MOLECULE TYPE:peptide
   SEQUENCE: 
Ala Ala Ile Ala Thr Ile Lys Thr Lys Arg
1               5                   10
SEQ ID NO:45
   SEQUENCE LENGTH:10
   SEQUENCE TYPE:amino acid
   MOLECULE TYPE:peptide
   SEQUENCE: 
Ser Ile Gln Phe Val Asp Trp Cys Pro Thr
1               5                   10
SEQ ID NO:46
   SEQUENCE LENGTH:10
   SEQUENCE TYPE:amino acid
   MOLECULE TYPE:peptide
   SEQUENCE: 
Gly Phe Lys Tyr Val Gly Ile Asn Tyr Gln
1               5                   10
SEQ ID NO:47
   SEQUENCE LENGTH:10
   SEQUENCE TYPE:amino acid
   MOLECULE TYPE:peptide
   SEQUENCE: 
Pro Pro Thr Val Val Pro Gly Gly Asp Leu
1               5                   10
SEQ ID NO:48
   SEQUENCE LENGTH:10
   SEQUENCE TYPE:amino acid
   MOLECULE TYPE:peptide
   SEQUENCE: 
Ala Lys Val Pro Arg Ala Val Cys Met Leu
1               5                   10
SEQ ID NO:49
   SEQUENCE LENGTH:10
   SEQUENCE TYPE:amino acid
   MOLECULE TYPE:peptide
   SEQUENCE: 
Ser Asn Thr Thr Ala Ile Ala Glu Ala Trp
1               5                   10
SEQ ID NO:50
   SEQUENCE LENGTH:10
   SEQUENCE TYPE:amino acid
   MOLECULE TYPE:peptide
   SEQUENCE: 
Ala Arg Leu Asp His Lys Phe Asp Leu Met
1               5                   10
SEQ ID NO:51
   SEQUENCE LENGTH:10
   SEQUENCE TYPE:amino acid
   MOLECULE TYPE:peptide
   SEQUENCE: 
Tyr Ala Lys Arg Ala Phe Val His Trp Tyr
1               5                   10
SEQ ID NO:52
   SEQUENCE LENGTH:10
   SEQUENCE TYPE:amino acid
   MOLECULE TYPE:peptide
   SEQUENCE: 
Val Gly Glu Gly Met Glu Glu Gly Glu Phe
1               5                   10
SEQ ID NO:53
   SEQUENCE LENGTH:10
   SEQUENCE TYPE:amino acid
   MOLECULE TYPE:peptide
   SEQUENCE: 
Ser Glu Ala Arg Glu Asp Leu Ala Ala Leu
1               5                   10
SEQ ID NO:54
   SEQUENCE LENGTH:10
   SEQUENCE TYPE:amino acid
   MOLECULE TYPE:peptide
   SEQUENCE: 
Glu Lys Asp Tyr Glu Glu Val Gly Val Asp
1               5                   10
SEQ ID NO:55
   SEQUENCE LENGTH:12
   SEQUENCE TYPE:amino acid
   MOLECULE TYPE:peptide
   SEQUENCE: 
Ser Met Glu Asp Asn Gly Glu Glu Gly Asp Glu Tyr
1               5                   10
SEQ ID NO:56
   SEQUENCE LENGTH:15
   SEQUENCE TYPE:amino acid
   MOLECULE TYPE:peptide
   SEQUENCE: 
Val His Glu Ile Arg Gly His Gln Phe Val Ala Thr Phe Phe Arg
1               5                   10                  15
SEQ ID NO:57
   SEQUENCE LENGTH:15
   SEQUENCE TYPE:amino acid
   MOLECULE TYPE:peptide
   SEQUENCE: 
Gln Pro His Phe Cys Ser Leu Cys Ser Asp Phe Met Trp Gly Leu
1               5                   10                  15
SEQ ID NO:58
   SEQUENCE LENGTH:15
   SEQUENCE TYPE:amino acid
   MOLECULE TYPE:peptide
   SEQUENCE: 
Asn Lys Gln Gly Tyr Gln Cys Gln Leu Cys Ser Ala Ala Val His
1               5                   10                  15
SEQ ID NO:59
   SEQUENCE LENGTH:15
   SEQUENCE TYPE:amino acid
   MOLECULE TYPE:peptide
   SEQUENCE: 
Lys Lys Cys His Glu Lys Val Ile Met Gln Cys Pro Gly Ser Ala
1               5                   10                  15
SEQ ID NO:60
   SEQUENCE LENGTH:15
   SEQUENCE TYPE:amino acid
   MOLECULE TYPE:peptide
   SEQUENCE: 
Lys Asn Thr Lys Glu Thr Met Ala Leu Lys Glu Arg Phe Lys Val
1               5                   10                  15
SEQ ID NO:61
   SEQUENCE LENGTH:15
   SEQUENCE TYPE:amino acid
   MOLECULE TYPE:peptide
   SEQUENCE: 
Asp Ile Pro His Arg Phe Lys Thr Tyr Asn Phe Lys Ser Pro Thr
1               5                   10                  15
SEQ ID NO:62
   SEQUENCE LENGTH:15
   SEQUENCE TYPE:amino acid
   MOLECULE TYPE:peptide
   SEQUENCE: 
Phe Cys Asp His Cys Gly Ser Met Leu Tyr Gly Leu Phe Lys Gln
1               5                   10                  15
SEQ ID NO:63
   SEQUENCE LENGTH:15
   SEQUENCE TYPE:amino acid
   MOLECULE TYPE:peptide
   SEQUENCE: 
Gly Leu Arg Cys Glu Val Cys Asn Val Ala Cys His His Lys Cys
1               5                   10                  15
SEQ ID NO:64
   SEQUENCE LENGTH:15
   SEQUENCE TYPE:amino acid
   MOLECULE TYPE:peptide
   SEQUENCE: 
Glu Arg Leu Met Ser Asn Leu Cys Gly Val Asn Gln Lys Gln Leu
1               5                   10                  15
SEQ ID NO:65
   SEQUENCE LENGTH:15
   SEQUENCE TYPE:amino acid
   MOLECULE TYPE:peptide
   SEQUENCE:

Asn Lys Gln Glu Tyr Gln Cys Gln Leu Cys Ser Ala Ala Val His
1               5                   10                  15

## Claims

1. An aligned peptide array comprising, as separate elements, a plurality of peptide segments obtained by dividing the amino acid sequence of a protein into non-overlapping sequence segments of equal length and of any suitable frame and synthesizing peptides on the basis of said sequence segments, wherein the peptide segments maintain each position thereof.

2. An aligned peptide array comprising, as separate elements, a plurality of peptide segments obtained by dividing the amino acid sequence of a protein into non-overlapping sequence segments of equal length and of any suitable frame and synthesizing peptides on the basis of said sequence segments, the amino acid sequences of said peptide segments expressing the amino acid sequence of said protein, wherein the peptide segments maintain each position thereof.

3. An immobilized aligned peptide array preparation obtained by immobilizing the aligned peptide array of claim 1 or 2.

4. An aligned peptide array membrane comprising a membrane on which the aligned peptide array of claim 1 or 2 is immobilized.

5. An immobilized aligned peptide array preparation comprising a gel on which the aligned peptide array of claim 1 or 2 is immobilized.

6. An aligned peptide array plate comprising a gel in the form of a plate on which the aligned peptide array of claim 1 or 2 is immobilized.

7. An aligned peptide array preparation, comprising a series of microtiter wells in which solutions containing the peptide segments constituting the aligend peptide array of claim 1 or 2 are placed in such a way that they exist separately from each other.

8. A microchip comprising an integrated circuit on which the aligned peptide array of claim 1 or 2 is immoblizied.

9. A microdevice comprising an integrated circuit on which the aligned peptide array of claim 1 or 2 is immobilized.

10. A method for the detection of a binding or interaction site of a protein which comprises detecting the binding or interaction site of any suitable protein or a specific protein with a ligand therefore by using the aligned peptide array of claim 1 or 2 or an immobilized aligned peptide array preparation derived therefrom.

11. A method for the detection of a binding or interaction site of a protein as claimed in claim 10 wherein the ligand is an antibody.

12. A method for the detection of a binding or interaction site of a protein as claimed in claim 10 wherein the ligand is a pharmaceutical drug, an agricultural chemical, or an insecticide.

13. A method for the detection of a binding or interaction site of a protein as claimed in claim 10 wherein the ligand is a pheromone or a hormone.

14. A method for the detection of a binding or interaction site of a protein as claimed in claim 10 wherein the ligand is another protein.

15. A method for the detection of a binding or interaction site of a protein as claimed in claim 10 wherein the ligand is a nucleic acid.

16. A method for the detection of a binding or interaction site of a protein as claimed in claim 10, wherein the ligand is a carbohydrate.

17. A method for the detection of a binding or interaction site of a protein as claimed in claim 10, wherein the ligand is a lipid.

18. A method for the detection of a ligand for a protein which comprises a step using the detection method of claim 10.

19. A method for the modification of a protein which comprises a step using the detection method of claim 10.

20. A method for the design of a protein which comprises a step using the detection method of claim 10.

21. A method for the modification of a ligand detected by the detection method of claim 18 which comprises a step utilizing the information on a binding or interaction site of a protein as obtained by the detection method of claim 10.

22. A method for the design of a ligand detected by the detection method of claim 18 which comprises a step utilizing the information on a binding or interaction site of a protein as obtained by the detection method of claim 10.

23. An immunoassay method which comprises using the aligned peptide array of claim 1 or 2 or an immobilized aligned peptide array preparation derived therefrom.

24. An enzyme immunoassay method, represented by ELISA, which comprises using the aligned peptide array of claim 1 or 2 or an immobilized aligned peptide array preparation derived therefrom.

25. A viroimmunoassay method which comprises using the aligned peptide array of claim 1 or 2 or an immobilized aligned peptide array preparation derived therefrom.

26. A metalloimmunoassay method which comprises using the aligned peptide array of claim 1 or 2 or an immobilized aligned peptide array preparation derived therefrom.

27. A fluoroimmunoassay method which comprises using the aligned peptide array of claim 1 or 2 or an immobilized aligned peptide array preparation derived therefrom.

28. A radioimmunoassay method which comprises using the aligned peptide array of claim 1 or 2 or an immobilized aligned peptide array preparation derived therefrom.

## Patentansprüche

1. Gerichtete Peptidanordnung, die als getrennte Elemente mehrere Peptidsegmente enthält, die dadurch gewonnen wurden, daß man die Aminosäuresequenz eines Proteins in nichtüberlappende Sequenzsegmente mit gleicher Länge und einem geeigneten Rahmen teilte und Peptide synthetisierte, die auf diesen Sequenzsegmenten beruhen, wobei die Peptidsegmente jede Position davon einnehmen.

2. Gerichtete Peptidanordnung, die als getrennte Elemente mehrere Peptidsegmente enthält, die dadurch gewonnen wurden, daß man die Aminosäuresequenz eines Proteins in nichtüberlappende Sequenzsegmente mit gleicher Länge und einem geeigneten Rahmen teilte und Peptide synthetisierte, die auf diesen Sequenzsegmenten beruhen, wobei die Aminosäuresequenzen der Peptidsegmente die Aminosäuresequenz des Proteins ausdrücken, wobei die Peptidsegmente jede Position davon einnehmen.

3. Immobilisiertes gerichtetes Peptidanordnungspräparat, das dadurch gewonnen wurde, daß man die gerichtete Peptidanordnung nach Anspruch 1 oder 2 immobilisierte.

4. Gerichtete Peptidanordnungsmembran mit einer Membran, auf der die gerichtete Peptidanordnung nach Anspruch 1 oder 2 immobilisiert ist.

5. Immobilisiertes gerichtetes Peptidanordnungspräparat mit einem Gel, auf dem die gerichtete Peptidanordnung nach Anspruch 1 oder 2 immobilisiert ist.

6. Gerichtete Peptidanordnungsplatte mit einem Gel in der Form einer Platte, auf dem die gerichtete Peptidanordnung nach Anspruch 1 oder 2 immobilisiert ist.

7. Gerichtetes Peptidanordnungspräparat mit einer Reihe von Mikrotiternäpfchen, in die Lösungen, die die Peptidsegmente, die die gerichtete Peptidanordnung nach Anspruch 1 oder 2 darstellen, enthalten, so gegeben werden, daß sie getrennt voneinander vorliegen.

8. Mikrochip mit integriertem Schaltkreis, auf dem die gerichtete Peptidanordnung nach Anspruch 1 oder 2 immobilisiert ist.

9. Mikrogerät mit integriertem Schaltkreis, auf dem die gerichtete Peptidanordnung nach Anspruch 1 oder 2 immobilisiert ist.

10. Verfahren zum Nachweisen einer Bindungs- oder Interaktionsstelle eines Proteins, bei dem man die Bindungs- oder Interaktionsstelle eines beliebigen geeigneten Proteins oder eines spezifischen Proteins mit einem Liganden hierfür dadurch nachweist, daß man die gerichtete Peptidanordnung nach Anspruch 1 oder 2 oder ein davon abgeleitetes immobilisiertes gerichtetes Peptidanordnungspräparat verwendet.

11. Verfahren zum Nachweisen einer Bindungs- oder Interaktionsstelle eines Proteins nach Anspruch 10, wobei es sich bei dem Liganden um einen Antikörper handelt.

12. Verfahren zum Nachweisen einer Bindungs- oder Interaktionsstelle eines Proteins nach Anspruch 10, wobei es sich bei dem Liganden um einen pharmazeutischen Arzneistoff, eine Agrarchemikalie oder ein Insektizid handelt.

13. Verfahren zum Nachweisen einer Bindungs- oder Interaktionsstelle eines Proteins nach Anspruch 10, wobei es sich bei dem Liganden um ein Pheromon oder ein Hormon handelt.

14. Verfahren zum Nachweisen einer Bindungs- oder Interaktionsstelle eines Proteins nach Anspruch 10, wobei es sich bei dem Liganden um ein anderes Protein handelt.

15. Verfahren zum Nachweisen einer Bindungs- oder Interaktionsstelle eines Proteins nach Anspruch 10, wobei es sich bei dem Liganden um eine Nukleinsäure handelt.

16. Verfahren zum Nachweisen einer Bindungs- oder Interaktionsstelle eines Proteins nach Anspruch 10, wobei es sich bei dem Liganden um ein Kohlenhydrat handelt.

17. Verfahren zum Nachweisen einer Bindungs- oder Interaktionsstelle eines Proteins nach Anspruch 10, wobei es sich bei dem Liganden um ein Lipid handelt.

18. Verfahren zum Nachweisen eines Liganden für ein Protein, das einen Schritt umfaßt, bei dem das Nachweisverfahren nach Anspruch 10 verwendet wird.

19. Verfahren zur Modifizierung eines Proteins, das einen Schritt umfaßt, bei dem das Nachweisverfahren von Anspruch 10 verwendet wird.

20. Verfahren zum Konstruieren eines Proteins, das einen Schritt umfaßt, bei dem das Nachweisverfahren von Anspruch 10 verwendet wird.

21. Verfahren zur Modifizierung eines mit dem Nachweisverfahren nach Anspruch 18 nachgewiesenen Liganden, das einen Schritt umfaßt, bei dem man die durch das Nachweisverfahren nach Anspruch 10 erhaltene Information über eine Bindungs- oder Interaktionsstelle eines Proteins verwendet.

22. Verfahren zum Konstruieren eines mit dem Nachweisverfahren nach Anspruch 18 nachgewiesenen Liganden, das einen Schritt umfaßt, bei dem man die durch das Nachweisverfahren nach Anspruch 10 erhaltene Information über eine Bindungs- oder Interaktionsstelle eines Proteins verwendet.

23. Immunassay-Verfahren, bei dem man die gerichtete Peptidanordnung nach Anspruch 1 oder 2 oder ein davon abgeleitetes immobilisiertes gerichtetes Peptidanordnungspräparat verwendet.

24. Ein durch ELISA repräsentiertes EnzymImmunassay-Verfahren, bei dem man die gerichtete Peptidanordnung nach Anspruch 1 oder 2 oder ein davon abgeleitetes immobilisiertes gerichtetes Peptidanordnungspräparat verwendet.

25. Virus-Immunassay-Verfahren, bei dem man die gerichtete Peptidanordnung nach Anspruch 1 oder 2 oder ein davon abgeleitetes immobilisiertes gerichtetes Peptidanordnungspräparat verwendet.

26. Metall-Immunassay-Verfahren, bei dem man die gerichtete Peptidanordnung nach Anspruch 1 oder 2 oder ein davon abgeleitetes immobilisiertes gerichtetes Peptidanordnungspräparat verwendet.

27. Fluoreszenz-Immunassay-Verfahren, bei dem man die gerichtete Peptidanordnung nach Anspruch 1 oder 2 oder ein davon abgeleitetes immobilisiertes gerichtetes Peptidanordnungspräparat verwendet.

28. Radio-Immunassay-Verfahren, bei dem man die gerichtete Peptidanordnung nach Anspruch 1 oder 2 oder ein davon abgeleitetes immobilisiertes gerichtetes Peptidanordnungspräparat verwendet.

## Revendications

1. Réseau peptidique aligné comprenant, en tant qu'éléments distincts, une pluralité de segments peptidiques obtenus en divisant la séquence aminoacide d'une protéine en segments de séquence ne se chevauchant pas de longueur égale et de quelconque structure appropriée et en synthétisant des peptides sur la base desdits segments de séquence, dans lequel les segments peptidiques maintiennent chaque position de ce dernier.

2. Réseau peptidique aligné comprenant, en tant qu'éléments distincts, une pluralité de segments peptidiques obtenus en divisant la séquence aminoacide d'une protéine en segments de séquence ne se chevauchant pas de longueur égale et de quelconque structure appropriée et en synthétisant des peptides sur la base desdits segments de séquence, les séquences aminoacide desdits segments peptidiques exprimant la séquence aminoacide de ladite protéine, dans lequel les segments peptidiques maintiennent chaque position de ce dernier.

3. Préparation de réseau peptidique aligné immobilisé obtenue en immobilisant le réseau peptidique aligné selon la revendication 1 ou 2.

4. Membrane de réseau peptidique aligné comprenant une membrane sur laquelle le réseau peptidique aligné selon la revendication 1 ou 2 est immobilisé.

5. Préparation de réseau peptidique aligné immobilisé comprenant un gel sur lequel le réseau peptidique aligné selon la revendication 1 ou 2 est immobilisé.

6. Plaque de réseau peptidique aligné immobilisé comprenant un gel sous la forme d'une plaque sur laquelle le réseau peptidique aligné selon la revendication 1 ou 2 est immobilisé.

7. Préparation de réseau peptidique aligné, comprenant une série de récipients de microtitrage dans lesquels des solutions contenant les segments peptidiques constituant le réseau peptidique aligné selon la revendication 1 ou 2 sont placées de sorte qu'elles existent distinctement les unes des autres.

8. Micropuce comprenant un circuit intégré sur lequel le réseau peptidique aligné selon la revendication 1 ou 2 est immobilisé.

9. Microcomposant comprenant un circuit intégré sur lequel le réseau peptidique aligné selon la revendication 1 ou 2 est immobilisé.

10. Procédé pour le dépistage d'une liaison ou d'un site d'interaction d'une protéine qui comprend le dépistage d'une liaison ou d'un site d'interaction d'une quelconque protéine appropriée ou d'une protéine spécifique par un ligand en utilisant pour cela le réseau peptidique aligné selon la revendication 1 ou 2 ou une préparation de réseau peptidique aligné immobilisé obtenue à partir de ce dernier.

11. Procédé pour le dépistage d'une liaison ou d'un site d'interaction d'une protéine selon la revendication 10, dans lequel le ligand est un anticorps.

12. Procédé pour le dépistage d'une liaison ou d'un site d'interaction d'une protéine selon la revendication 10, dans lequel le ligand est un produit pharmaceutique, un produit chimique agricole ou un insecticide.

13. Procédé pour le dépistage d'une liaison ou d'un site d'interaction d'une protéine selon la revendication 10, dans lequel le ligand est une phéromone ou une hormone.

14. Procédé pour le dépistage d'une liaison ou d'un site d'interaction d'une protéine selon la revendication 10, dans lequel le ligand est une autre protéine.

15. Procédé pour le dépistage d'une liaison ou d'un site d'interaction d'une protéine selon la revendication 10, dans lequel le ligand est un acide nucléique.

16. Procédé pour le dépistage d'une liaison ou d'un site d'interaction d'une protéine selon la revendication 10, dans lequel le ligand est un hydrate de carbone.

17. Procédé pour le dépistage d'une liaison ou d'un site d'interaction d'une protéine selon la revendication 10, dans lequel le ligand est un lipide.

18. Procédé pour le dépistage d'un ligand pour une protéine qui comprend une étape utilisant le procédé de dépistage selon la revendication 10.

19. Procédé pour la modification d'une protéine qui comprend une étape utilisant le procédé de dépistage selon la revendication 10.

20. Procédé pour la conception d'une protéine qui comprend une étape utilisant le procédé de dépistage selon la revendication 10.

21. Procédé pour la modification d'un ligand dépisté par le procédé de dépistage selon la revendication 18 qui comprend une étape utilisant les informations concernant une liaison ou un site d'interaction d'une protéine telles qu'obtenues par le procédé de dépistage selon la revendication 10.

22. Procédé pour la conception d'un ligand dépisté par le procédé de dépistage selon la revendication 18 qui comprend une étape utilisant les informations concernant une liaison ou un site d'interaction d'une protéine telles qu'obtenues par le procédé de dépistage selon la revendication 10.

23. Procédé d'immuno-essai qui comprend l'utilisation du réseau peptidique aligné selon la revendication 1 ou 2 ou d'une préparation de réseau peptidique aligné immobilisé obtenue à partir de ce dernier.

24. Procédé d'immuno-essai enzymologique, représenté par ELISA, qui comprend l'utilisation du réseau peptidique aligné selon la revendication 1 ou 2 ou d'une préparation de réseau peptidique aligné immobilisé obtenue à partir de ce dernier.

25. Procédé de viro-immuno-essai, qui comprend l'utilisation du réseau peptidique aligné selon la revendication 1 ou 2 ou d'une préparation de réseau peptidique aligné immobilisé obtenue à partir de ce dernier.

26. Procédé de métallo-immuno-essai, qui comprend l'utilisation du réseau peptidique aligné selon la revendication 1 ou 2 ou d'une préparation de réseau peptidique aligné immobilisé obtenue à partir de ce dernier.

27. Procédé de fluoro-immuno-essai, qui comprend l'utilisation du réseau peptidique aligné selon la revendication 1 ou 2 ou d'une préparation de réseau peptidique aligné immobilisé obtenue à partir de ce dernier.

28. Procédé de radio-immuno-essai, qui comprend l'utilisation du réseau peptidique aligné selon la revendication 1 ou 2 ou d'une préparation de réseau peptidique aligné immobilisé obtenue à partir de ce dernier.
